# EUROPÄISCHE PATENTSCHRIFT

(11) **EP 1 432 522 B1**
(45) Veröffentlichungstag und Bekanntmachung des Hinweises auf die Patenterteilung: **06.07.2005**
(21) Anmeldenummer: 02781092.8
(22) Anmeldetag: 18.09.2002
(51) Int. Cl.: B05B 1/00, A61B 17/56

(54) **APPLIKATOR**
APPLICATOR
APPLICATEUR

(30) Priorität: 19.09.2001 DE 10146303
(43) Veröffentlichungstag der Anmeldung: 30.06.2004
(73) Patentinhaber: Marx, Rudolf, 53533 Eichenbach (DE); Fischer, Horst, 52074 Aachen (DE); Niethard, Fritz Uwe, 52076 Aachen (DE); Wirtz, Dieter Christian, 52072 Aachen (DE)
(72) Erfinder: Marx, Rudolf, 53533 Eichenbach (DE); Fischer, Horst, 52074 Aachen (DE); Niethard, Fritz Uwe, 52076 Aachen (DE); Wirtz, Dieter Christian, 52072 Aachen (DE)
(74) Vertreter: Jostarndt, Hans-Dieter, Dr. Dipl.-Phys.
(86) Internationale Anmeldenummer: PCT/DE2002/003470
(87) Internationale Veröffentlichungsnummer: WO 2003/024607

(56) Entgegenhaltungen:
- EP-A- 0 485 653
- DE-U- 29 517 958
- GB-A- 1 586 089
- US-A- 4 673 353
- US-B1- 6 305 934

## Beschreibung

Die Erfindung betrifft einen Applikator zum Aufbringen von Substanzen und Schichten auf eine Oberfläche.

Das deutsche Gebrauchsmuster 295 17 958 offenbart ein Gerät für den inkrementalen Aufbau einer Zahnfüllung mit einem Handstück mit einem Ausbringröhrchen zum Aufbringen von Füllmaterial und einer Strahlungsquelle zum Aushärten des Füllmaterials, siehe Oberbegriff von Anspruch 1.

Die europäische Patentanmeldung 0 485 653 A1 zeigt ein Gerät, welches geeignet ist, Rohre von innen mit Füllmaterial bzw. Dichtmaterial zu verkleiden. Bei dieser Vorrichtung drückt ein sich in dem Rohr bewegender Kolben eine auszuhärtende Masse an die Innenwand und härtet sie mit einer Strahlungsquelle aus.

Die Schrift US 4,673,353 offenbart eine Vorrichtung, mit der man lichthärtende Materialien für den Dentalbereich applizieren kann.

Bei der Behandlung von Oberflächen besteht häufig der Bedarf, die Oberfläche mit Schichten von Polymeren zu beaufschlagen. An diese Schichten werden dann auch bestimmte Anforderungen gestellt. So soll die Schicht bestimmte chemische Eigenschaften haben, zum Beispiel als Haftvermittler dienen oder biokompatibel sein, eine bestimmte Schichtdicke aufweisen oder schnell aushärten.

In einigen Fällen besteht auch das Problem, Hohlräume von innen zu beschichten. Diese Hohlräume können höhlenförmig, rund, aber auch zylinderförmig, wie beispielsweise Innenoberflächen von Rohren oder sogar im medizinischen Bereich von Knochen sein.

Es ist die Aufgabe der Erfindung, eine Vorrichtung zu schaffen, mit denen Oberflächen, insbesondere Innenoberflächen beschichtet werden können.

Ausgehend vom Oberbegriff des Anspruchs 1 wird die Aufgabe dadurch gelöst, dass ein Applikator eingesetzt wird, der über einen Benetzungsring verfügt.

Vorteilhafte Weiterbildungen der Erfindung sind in den Unteransprüchen angegeben.

Im Folgenden soll die Erfindung beispielhaft beschrieben werden.

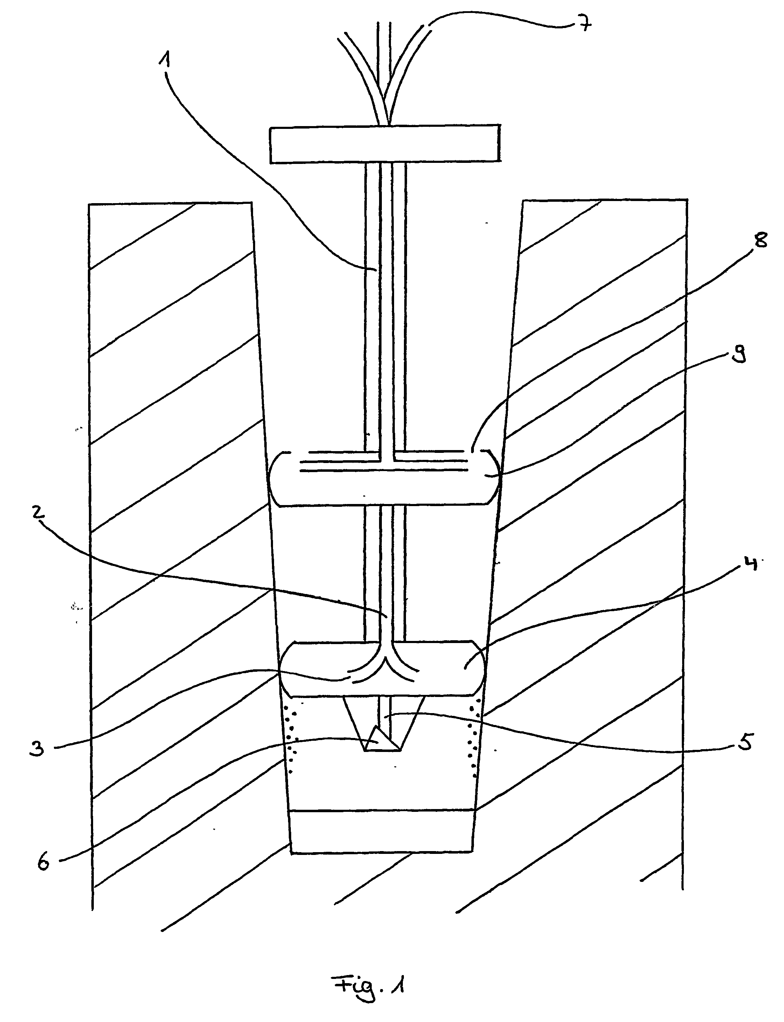

Die Figur 1 zeigt eine bevorzugte Ausführungsform des Applikators, bei dem durch einen Zylinder 1 eine Zuleitung 2 für Substanzen, eine Absaugung 7 und ein Lichtleiter 5 in den vorderen Bereich geführt werden, der in der Figur unten abgebildet ist. Am vorderen Ende befinden sich Austrittsöffnungen 3 für die Substanzen, welche durch einen Benetzungsring 4 austreten. Unterhalb des Benetzungsringes 4 befindet sich eine optische Vorrichtung 6 (z.B. ein Prisma oder Spiegel), durch welches das Licht des Lichtleiters 5 austritt. Oberhalb der Austrittsöffnung 3 ist eine Absaugöffnung 8 angeordnet, deren Öffnungen an der Oberfläche eines Abstreifringes 9 heraustreten.

Der in Figur 1 abgebildete Applikator ist stabförmig ausgebildet. In diesem Beispiel ist der Stab ein Zylinder 1, der einen Hohlraum aufweist. Am oberen Ende des Zylinders 1 tritt mindestens.eine Zuleitung 2 in den Innenraum des Zylinders 1 ein, die am unteren Ende des Zylinders 1 in mindestens eine Austrittsöffnung 3 mündet. Durch diese Zuleitung 2 kann mindestens eine photochemisch polymerisierende Substanz zur Austrittsöffnung 3 geleitet werden. In einer weiteren Ausführungsform der Erfindung können auch zwei, drei oder noch mehr Zuleitungen 2,2a,2b,2c,2d.... für Substanzen vorhanden sein, die von einem oder mehreren Reservoirs gespeist werden. Die Substanzen können durch Pumpeinheiten, die in der Figur nicht dargestellt sind, gefördert werden. Als Pumpeinheiten kommen beispielsweise mit Ventilen ausgestattete Gummibälle in Betracht. Dabei können auch für die Vorbehandlung der Oberfläche entsprechende Stoffe, wie beispielsweise Säuren, gefördert werden. Jede der Zuleitungen 2 kann in eine oder mehrere Austrittsöffnungen 3,3a,3b..... münden. Vorzugsweise mündet jede Zuleitung 2 in mehrere, zum Beispiel 5,6,7, oder 8 Austrittsöffnungen 3,3a,3b,3c,3d..., die vorzugsweise vom Zentrum des zylinderförmigen Stabes im Wesentlichen radial nach außen gerichtet und im Wesentlichen gleichen Winkel zueinander angeordnet sind. Dies ist insbesondere für die Innenbeschichtung von rohrförmigen Körpern von Vorteil. Die Austrittsöffnungen 3,3a,3b können jede beliebige Form, wie einfache Öffnungen oder Schlauchaustritte annehmen, jedoch ist eine Düsenform bevorzugt, welche die austretenden Substanzen auch noch zerstäuben und somit für eine besonders gleichmäßige Beschichtung sorgen. Die Austrittsöffnungen 3,3a,3b...können sternförmig durch kleine Röhrchen von dem Zylinder 1 ausgehen. Der Druck, mit dem die meist flüssigen Substanzen aus den Austrittsöffnungen 3 austritt, beträgt vorzugsweise 0,05 bis 2,0 bar. Besonders bevorzugt sind 0,2 bis 1,0 bar. Zur Unterstützung der Beschichtung kann ein ringförmiger Körper, wie in Figur 1 dargestellt, als Benetzungsring 4 fungieren. In dieser Ausführungsform enden die Zuleitungen 2,2a,2b,... im Benetzungsring 4 und gehen in die Austrittsöffnungen 3,3a,3b,.. über, welche sich auf der Oberfläche des Benetzungsringes 4 befinden. Die Austrittsöffnungen 3,3a,3b,... befinden sich vorzugsweise auf dem Außenumfang des ringförmigen Körpers, da dort auch die beste Verteilung der austretenden Stoffe durch diesen Benetzungsring 4 stattfindet. Die Austrittsöffnungen 3 können sich jedoch auch auf der oberen oder unteren Seite des ringförmigen Körpers befinden. Der Benetzungsring 4 besteht vorzugsweise mindestens teilweise aus elastischem Material. Es können jedoch andere Materialien, wie Weich- oder Hartgummi oder ein poröser Gummi (z.B. Moosgummi) oder Silikone eingesetzt werden. In einer besonders bevorzugten Ausführungsform besteht der Benetzungsring wenigstens in seinem Außenbereich aus biokompatiblem Material. Dies ermöglicht zum Beispiel den Einsatz des Applikators bei medizinischen Operationen, insbesondere bei der Beschichtung des Innenraumes von Knochen. Als biokompatible Materialien kommen beispielsweise Werkstoffe auf Silikonbasis in Betracht. Durch Hin- und Herschieben des Applikators oder auch nur durch Ziehen in eine Richtung kann die aufgetragene Substanz mittels des Benetzungsringes 4 gleichmäßig verteilt werden. Der Benetzungsring 4 hat vorzugsweise einen Durchmesser von 10 bis 60 mm. Je nach Anforderung kann der Benetzungsring 4 auch auswechselbar angebracht sein und verschiedene Durchmesser aufweisen.

Erfindungsgemäß ist der Applikator weiterhin mit Mitteln zum Eintrag von elektromagnetischer Strahlung, insbesondere sichtbarem und UV-Licht ausgestattet. Dem Applikator ist entweder fest verbunden oder frei zuschaltbar eine Strahlungsquelle zugeordnet, welche beispielsweise Licht der Wellenlänge 380,300,254 oder 248 nm ausstrahlt. Besonders bevorzugt ist ein Wellenlängenbereich in der Größenordnung von 380 nm, jedoch kann jede Wellenlänge eingesetzt werden, die zur Photopolymerisation der entsprechenden Substanzen geeignet ist. Als Strahlungsquellen kommen beispielsweise Xenon-, Xenonhochdrucklampen oder Quecksilberdampflampen oder Laser der gewünschten Wellenlänge in Betracht. Entsprechend kann jede Strahlungsquelle eingesetzt werden, welche zur Erzeugung von elektromagnetischer Strahlung geeignet ist, die eine Photopolymerisation von Substanzen bewirkt. Die jeweilige erforderliche Wellenlänge richtet sich nach dem Absorptions- und Aktivierungsverhalten der Substanzen. Die Strahlungsquelle, die in der Figur nicht dargestellt ist, steht mit Lichtleitern 5 in Verbindung, welche die Strahlung durch den Zylinder 1 einem Prisma 6 oder einer entsprechenden optischen Vorrichtung zuführen. Die Lichtleiter bestehen vorzugsweise aus Glasfasern, besonders bevorzugt aus Quarzglasfasern, wenn UV-Licht weitergeleitet werden soll.
Die Lichtaustrittsstelle, die vorzugsweise mit dem Prisma 6 oder einem Quarzprisma ausgestattet ist, befindet sich im Bereich der Austrittsstellen für die zu polymerisierenden Stoffe. Das Prisma oder die entsprechende optische Vorrichtung kann auch aus einem geeigneten Kunststoff hergestellt sein. Mit Austrittsstelle ist jede Position gemeint, die geeignet ist, genügend Licht an die Stellen zu strahlen, um eine Polymerisation der Substanzen auf der zu beschichtenden Oberfläche zu bewirken. Vorzugsweise beträgt der Abstand zwischen den Austrittsöffnungen 3 und der Lichtaustrittsstelle 5 bis 200 mm.Je nachdem, ob das Licht durch das bevorzugte Prisma 6 oder austretende Glasfasern auf die auszuhärtenden Bereiche gelenkt wird, kann der Austritt direkt an dem Benetzungsring 4, zwischen einzelnen Austrittsdüsen, für den Fall, dass die Stoffe durch eine sternförmige Düsenanordnung aufgebracht wird, oder auch unterhalb der Austrittsöffnungen angebracht sein. In einer wenig bevorzugten Ausführungsform kann das zur Photopolymerisation eingebrachte Licht auch ohne besondere Lenkung im unteren Bereich des Zylinders 1, nämlich in der Nähe der Austrittsöffnungen 3,3a,3b,3c..... diffus eingebracht werden, zum Beispiel durch einen Glas- oder Kunststoffkörper beliebiger Gestalt. In einer weiteren bevorzugten Weiterbildung der Erfindung ist der Applikator mit Mitteln zum Absaugen von Flüssigkeit ausgestattet. Hierzu ist eine Absaugleitung 7 vorgesehen, welche durch den Stab geführt wird und oberhalb bzw. hinter den Austrittsöffnungen 3 für den Auftrag der Substanzen endet. In einer einfachen Ausführungsform sind lediglich Absaugöffnungen 8 vorhanden, welche sich in dem Zylinder 1 befinden. Die Absaugöffnungen 8 können auch in Form von Röhrchen vorliegen, die aus dem Zylinder 1 austreten und die in der Figur nicht abgebildet sind. In einer ersten zweckmäßigen Ausführungsform ist mindestens eine Absaugöffnung 8 vorhanden. Vorzugsweise sind jedoch mehrere Absaugöffnungen 8 angebracht. In einer weiterhin bevorzugten Ausführungsform besitzt der Applikator einen Abstreifring 9, der sich oberhalb der Austrittsöffnung 3 befindet. Der Abstreifring 9 ist vorzugsweise wenigstens teilweise aus elastischem Material ausgebildet. Grundsätzlich können die gleichen Materialien wie beim Benetzungsring eingesetzt werden. In bevorzugten Ausführungsformen befinden sich die Absaugöffnungen 8 auf dem Außenradius des Abstreifringes 9 und/oder auf der dem Benetzungsring 4 abgewandten Seite.
In einer weiteren Ausgestaltung der Erfindung können die Absaugöffnungen 8 und die Austrittsöffnungen 3 in ihrem Längsabstand zueinander verstellbar und beweglich angebracht sein. Hierzu kann zum Beispiel eine verstellbare Verschraubung dienen.
Bei der hier beschriebenen Ausführungsform werden der Lichtleiter 5, die Zuleitungen 2 und die Absaugleitungen 7 im Zentrum des Zylinders 1 geführt. In einer weniger bevorzugten Ausführungsform ist es auch denkbar, diese Leitungen außerhalb des Stabes zu führen.
Die beschriebene Ausführungsform der Erfindung ist besonders gut geeignet für die Innenbeschichtung von Rohren oder die Behandlung von Aushöhlungen in Knochen, die aus medizinischen Gründen ausgefräst werden mussten.
In der allgemeinsten Form ist die erfindungsgemäße Vorrichtung als Kombination von Auftragemitteln für polymerisierbare chemische Komponenten und eine Strahlungsquelle für elektromagnetische Strahlung aufzufassen, die je nach Anwendungsgebiet unterschiedlich ausgestaltet sein kann. Für die Beschichtung einer im Wesentlichen glatten Oberfläche kann an Stelle eines Benetzungsringes 4 ein Düsenkamm und eine längliche Lichtaustrittsstelle treten. Der Applikator ist vorzugsweise stabförmig ausgebildet, jedoch können auch Abweichungen von einer geraden Geometrie vorteilhaft sein, zum Beispiel, wenn ein von innen zu beschichtendes Rohr gekrümmt ist.

### Beispiel:

### Medizinische Anwendung:

Der erfindungsgemäße Applikator kann beispielsweise in der Orthopädie zur Aufbringung einer Beschichtung in einen implantatspezifisch präparierten Knochen zur stabilen Verankerung einer Prothese eingesetzt werden. Ist eine alte Prothese zu ersetzen, so wird das alte Implantat aus dem Knochen, beispielsweise dem Femur, entfernt und die innere Oberfläche des so freigelegten, natürlichen Femurs behandelt, damit das neue Implantat eingesetzt werden kann. Hierzu kann ein Verfahren der Anmelder verwendet werden, das unter dem Aktenzeichen ACFMW 5101 PDE am selben Tag angemeldet worden ist, wie diese Anmeldung. Zu diesem Zweck wird durch die Zuleitung 2 eine schwache Säure, wie beispielsweise Zitronensäure, Maleinsäure oder Phosphorsäure den Austrittsstellen 3,3a,3b,3c... zugeführt und mit dem Benetzungsring 4 mit der Knochenoberfläche in Kontakt gebracht. Davor oder zeitgleich wird der Abstreifring 9 hin und her oder auch nur in eine Richtung bewegt und mittels der Absaugung Blut oder Körperflüssigkeit durch die Absaugöffnungen 8,8a,8b,8c.....abgezogen, um die Knochenoberfläche für die Benetzung mit der schwachen Säure vorzubereiten. Der Applikator wird dabei in Richtung des Abstreifringes 9 gezogen. In einem weiteren Schritt wird den Austrittsöffungen 3a,3b,3c... oder 3i,3j,3k durch eine andere Zuleitung 2a ein amphiphiles Molekül, wie 2-Hydroxyethylmetharcrylat zugeführt, welches wiederum mit dem Benetzungsring 4 auf der Innenoberfläche des Femurs verteilt wird. In einem weiteren Schritt wird durch eine weitere Zuleitung 2b ein photopolymerisierendes Monomer den Austrittsöffnungen 3a,3b,3c, 3i,3j,3k oder 3x,3y,3z zugeführt, mit dem Benetzungsring 4 verteilt und gleichzeitig mittels der Strahlungsquelle, dem Lichtleiter 5 und dem Prisma 6 mit elektromagnetischer Strahlung einer geeigneten Wellenlänge bestrahlt. Als photopolymerisierendes Monomer kommt beispielsweise ein Gemisch aus Bisphenolglycidylmethactrylat und Triethylenglycoldimethacrylat in Betracht. Die eingestrahlte Wellenlänge kann 300 nm betragen.
Die auf diese Weise präparierte beschichtete Innenoberfläche des Knochens, vorzugsweise dem Femur oder der Hüftpfanne, kann dann mit Knochenzement (PMMA) gefüllt werden, welcher die Prothese aufnimmt. Die mit dem Applikator aufgebrachte Schicht dient nun als Haftvermittler für das PMMA mit dem Knochen.

### Bezugszeichenliste:

- 1.: Zylinder
- 2.: Zuleitung
- 3.: Austrittsöffnungen
- 4.: Benetzungsring
- 5.: Lichtleiter
- 6.: Prisma
- 7.: Absaugung
- 8.: Absaugöffnung
- 9.: Abstreifring

## Patentansprüche

1. Medizinischer Applikator zum Aufbringen einer Schicht auf eine Oberfläche, der im wesentlichen als Stab ausgebildet ist, ein Mittel zur Erzeugung einer elektromagnetischen Strahlung aufweist und Mittel zum Aufbringen mindestens einer Substanz, umfassend mindestens eine Zuleitung (2) mit mindestens einer Austrittsstelle(3), umfasst,
**dadurch gekennzeichnet,**
**dass** sich im Bereich der Austrittsstelle (3) ein Benetzungsring (4) befindet, der den Stab umgibt.

2. Medizinischer Applikator nach Anspruch 1,
**dadurch gekennzeichnet,**
**dass** die Mittel zum Erzeugen einer elektromagnetischen Strahlung ein Prisma oder eine andere geeignete optische Vorrichtung (6), welche am Ende des Stabes angebracht ist und eine Zuleitung für elektromagnetische Strahlung umfassen, die die Strahlung entlang des Stabes von der Strahlungsquelle in das Prisma einbringen.

3. Medizinischer Applikator nach einem der Ansprüche 1 oder 2,
**dadurch gekennzeichnet,**
**dass** die Mittel zur Zuleitung der elektromagnetischen Strahlung Glasfasern sind.

4. Medizinischer Applikator nach einem der Ansprüche 1 bis 3,
**dadurch gekennzeichnet,**
**dass** die Mittel zum Aufbringen einer Substanz mindestens eine Zuleitung (2) umfassen, welche in Austrittsstellen (3) münden, die dem Prisma (6) benachbart sind.

5. Medizinischer Applikator nach Anspruch 4,
**dadurch gekennzeichnet,**
**dass** er drei Zuleitungen (2) besitzt, welche in Austrittsstellen (3) münden.

6. Medizinischer Applikator nach einem der Ansprüche 4 oder 5,
**dadurch gekennzeichnet,**
**dass** die Austrittsstellen (3) ausgehend vom Zentrum des Stabes im Wesentlichen radial nach außen gerichtet sind.

7. Medizinischer Applikator nach einem der Ansprüche 4 bis 6,
**dadurch gekennzeichnet,**
**dass** die Austrittsstellen (3) als Düsen, einfache Öffnungen oder Schlauchenden ausgebildet sind.

8. Medizinischer Applikator nach Anspruch 7,
**dadurch gekennzeichnet,**
**dass** sich die Austrittsstellen (3) im Benetzungsring(4) befinden.

9. Medizinischer Applikator nach Anspruch 7 oder 8,
**dadurch gekennzeichnet,**
**dass** der ringförmige Körper (4) wenigstens in seinem Randbereich aus elastischem Material besteht.

10. Medizinischer Applikator nach Anspruch 9,
**dadurch gekennzeichnet,**
**dass** das elastische Material ein geschäumtes Polymer ist.

11. Medizinischer Applikator nach einem der Ansprüche 1 bis 10,
**dadurch gekennzeichnet,**
**dass** er Mittel zum Absaugen umfasst, deren Absaugöffnungen (8) hinter den Austrittsöffnungen (3) angebracht sind.

12. Medizinischer Applikator nach einem der Ansprüche 1 bis 11,
**dadurch gekennzeichnet,**
**dass** er einen Abstreifring (9) besitzt.

13. Medizinischer Applikator nach Anspruch 11 und 12
**dadurch gekennzeichnet,**
**dass** sich der Abstreifring (9) zwischen den Absaugöffnungen (8) und den Austrittsöffnungen (3) befindet.

14. Medizinischer Applikator nach Anspruch 11 und 12,
**dadurch gekennzeichnet,**
**dass** sich die Absaugöffnungen (8) auf dem Abstreifring (9) befinden.

15. Medizinischer Applikator nach Anspruch 12 oder 14,
**dadurch gekennzeichnet,**
**dass** der Abstreifring (9) aus elastischem Material besteht.

16. Medizinischer Applikator nach Anspruch 12 bis 15,
**dadurch gekennzeichnet,**
**dass** der Abstreifring (9) mindestens teilweise aus Polyurethanschaum besteht.

17. Medizinischer Applikator nach einem der Ansprüche 12 bis 16,
**dadurch gekennzeichnet,**
**dass** er über Mittel verfügt, welche den Abstand zwischen den Austrittsöffnungen (3) und den Absaugöffnungen (8) verändern.

18. Medizinischer Applikator nach einem der Ansprüche 1 bis 17,
**dadurch gekennzeichnet,**
**dass** er eine Strahlungsquelle für sichtbares und/oder UV-Licht umfasst.

19. Medizinischer Applikator nach einem oder mehreren der vorhergehenden Ansprüche,
**dadurch gekennzeichnet,**
**dass** er ein Mittel zur Reinigung enthält.

20. Medizinischer Applikator nach einem oder mehreren der vorhergegangenen Ansprüche,
**dadurch gekennzeinet,**
dass er ein Mittel zur Absaugung und/oder Spülung enthält.

## Claims

1. A medical applicator for applying a layer onto a surface, said applicator being essentially configured as a stick, having means for generating electromagnetic radiation and means for applying at least one substance, comprising at least one supply line (2) with at least one outlet (3),
**characterized in that**
a wetting ring (4) that surrounds the stick is located in the area of the outlet (3).

2. The medical applicator according to Claim 1,
**characterized in that**
the means for generating electromagnetic radiation comprise a prism or another suitable optical device (6) that is attached to the end of the stick and a supply line for electromagnetic radiation, both of which serve to introduce the radiation along the stick from the source of radiation into the prism.

3. The medical applicator according to one of Claims 1 or 2,
**characterized in that**
the means for supplying the electromagnetic radiation are glass fibers.

4. The medical applicator according to one of Claims 1 to 3,
**characterized in that**
the means for applying a substance comprise at least one supply line (2) that opens up into outlets (3) that are adjacent to the prism (6).

5. The medical applicator according to Claim 4,
**characterized in that**
it has three supply lines (2) that open up into outlets (3).

6. The medical applicator according to one of Claims 4 or 5,
**characterized in that**
the outlets (3), starting at the center of the stick, are oriented essentially radially towards the outside.

7. The medical applicator according to one of Claims 4 to 6,
**characterized in that**
the outlets (3) are configured as nozzles, simple openings or hose ends.

8. The medical applicator according to Claim 7,
**characterized in that**
the outlets (3) are situated in the wetting ring (4).

9. The medical applicator according to Claim 7 or 8,
**characterized in that**
at least the edge area of the ring-shaped elements (4) is made of elastic material.

10. The medical applicator according to Claim 9,
**characterized in that**
the elastic material is a foamed polymer.

11. A medical applicator according to one of Claims 1 to 10,
**characterized in that**
it comprises means for suction, whose suction openings (8) are situated behind the outlets (3).

12. The medical applicator according to one of Claims 1 to 11,
**characterized in that**
it has a scraping ring (9).

13. The medical applicator according to Claims 11 and 12,
**characterized in that**
the scraping ring (9) is situated between the suction openings (8) and the outlets (3).

14. The medical applicator according to Claims 11 and 12,
**characterized in that**
the suction openings (8) are situated on the scraping ring (9).

15. The medical applicator according to Claim 12 or 14,
**characterized in that**
the scraping ring (9) is made of elastic material.

16. The medical applicator according to Claims 12 to 15,
**characterized in that**
the scraping ring (9) is made at least partially of polyurethane foam.

17. The medical applicator according to one of Claims 12 to 16,
**characterized in that**
it has means that change the distance between the outlets (3) and the suction openings (8).

18. The medical applicator according to one of Claims 1 to 17,
**characterized in that**
it comprises a source of radiation for visible and/or UV light.

19. The medical applicator according to one or more of the preceding claims,
**characterized in that**
it contains means for cleaning.

20. The medical applicator according to one or more of the preceding claims,
**characterized in that**
it contains means for suction and/or rinsing.

## Revendications

1. Un applicateur médical pour appliquer une couche sur une surface, qui est formé pour l'essentiel comme barre, présente un moyen pour la production d'une radiation électromagnétique et des moyens pour la mise d'au moins une substance, contenant au moins une conduite d'alimentation (2) avec au moins un endroit de sortie (3),
**caractérisé en ce que,**
il se trouve au secteur de l'endroit de sortie (3) un anneau de mouillage (4), qui entoure la barre.

2. Un applicateur médical selon la revendication 1,
**caractérisé en ce que**
les moyens pour la production d'une radiation électromagnétique contiennent un prisme ou un autre dispositif optique convenable (6), qui est fixé à l'autre bout de la barre et une conduite d'alimentation pour une radiation électromagnétique qui mettent la radiation le long de la barre lors de la source de radiation dans le prisme.

3. Un applicateur médical selon une des revendications 1 ou 2,
**caractérisé en ce que**
les moyens pour la conduite d'alimentation de la radiation électromagnétique sont des fibres de verre.

4. Un applicateur médical selon une des revendications 1 à 3,
**caractérisé en ce que**
les moyens pour la mise d'une substance contiennent au moins une conduite d'alimentation (2), qui se jette dans des endroits de sortie (3), qui sont avoisinants au prisme (6).

5. Un applicateur médical selon la revendication 4,
**caractérisé en ce que**
il possède trois conduites d'alimentation (2) qui se jètent dans des endroits de sortie (3).

6. Un applicateur médical selon une des revendications 4 ou 5,
**caractérisé en ce que**
les endroits de sortie (3) sont, départant du centre de la barre, orientés pour l'essentiel d'une façon radiale vers l'extérieur.

7. Un applicateur médical selon une des revendications 4 à 6,
**caractérisé en ce que**
les endroits de sortie (3) sont formés comme des filières, des ouvertures simples ou des extrémités de tuyau.

8. Un applicateur médical selon la revendication 7,
**caractérisé en ce que**
les endroits de sortie (3) se trouvent dans l'anneau de mouillage (4).

9. Un applicateur médical selon la revendication 7 ou 8,
**caractérisé en ce que**
le corps en forme d'anneau (4) se compose au moins à son secteur latérale d'un matériel élastique.

10. Un applicateur médical selon la revendication 9,
**caractérisé en ce que**
le matériel élastique est un polymère expansé.

11. Un applicateur médical selon une des revendications 1 à 10,
**caractérisé en ce que**
il comporte des moyens pour aspirer dont les ouvertures d'aspiration (8) sont placées derrière les orifices de sortie (3).

12. Un applicateur médical selon une des revendications 1 à 11,
**caractérisé en ce que**
il possède un anneau racleur (9).

13. Un applicateur médical selon la revendication 11 et 12,
**caractérisé en ce que**
l'anneau racleur (9) se trouve entre les ouvertures d'aspiration (8) et les orifices de sortie (3).

14. Un applicateur médical selon la revendication 11 et 12,
**caractérisé en ce que**
les ouvertures d'aspiration (8) se trouvent sur l'anneau racleur (9).

15. Un applicateur médical selon la revendication 12 ou 14,
**caractérisé en ce que**
l'anneau racleur (9) est composé d'un matériel élastique.

16. Un applicateur médical selon la revendication 12 à 15,
**caractérisé en ce que**
l'anneau racleur (9) est composé au moins partiellement d'une mousse de polyuréthane.

17. Un applicateur médical selon une des revendications 12 à 16,
**caractérisé en ce que**
il dispose des moyens, qui varient la distance entre les orifices de sortie (3) et les ouvertures d'aspiration (8) .

18. Un applicateur médical selon une des revendications 1 à 17,
**caractérisé en ce que**
il contient une source de radiation pour la lumière visible et/ ou ultraviolette.

19. Un applicateur médical selon une ou plusieurs des revendications précédentes,
**caractérisé en ce que**
il contient un moyen pour le nettoyage.

20. Un applicateur médical selon une ou plusieurs des revendications précédentes,
**caractérisé en ce que**
il contient un moyen pour l'aspiration et/ ou le rinçage.
